## Europäisches Patentamt
## European Patent Office
## Office européen des brevets

(11) Publication number: **0 013 133**
**B1**

(12) **EUROPEAN PATENT SPECIFICATION**

(45) Date of publication of patent specification: **12.01.83**

(51) Int. Cl.³: **C 07 C 37/78, C 07 C 39/07, C 07 C 39/06**

(21) Application number: **79302973.7**

(22) Date of filing: **19.12.79**

(54) **Process for separation of alkylphenols by azeotropic distillation.**

(30) Priority: **25.12.78 JP 158690/78**

(43) Date of publication of application:
**09.07.80 Bulletin 80/14**

(45) Publication of the grant of the patent:
**12.01.83 Bulletin 83/2**

(84) Designated Contracting States:
**DE FR GB**

(56) References cited:
**US - A - 3 337 424**

(73) Proprietor: **MITSUI PETROCHEMICAL INDUSTRIES, LTD.**
**2-5, Kasumigaseki 3-chome Chiyoda-ku Tokyo 100 (JP)**

(72) Inventor: **Takahata, Kazunori**
**2-6, Misono 1-chome**
**Ohtake-shi, Hiroshima-ken (JP)**
Inventor: **Taniguchi, Katsuo**
**2-4, Muronoki-cho 1-chome**
**Iwakuni-shi, Yamaguchi-ken (JP)**
Inventor: **Fujimoto, Tadaaki**
**1749, Shimokubara Niomaru Shuto-cho**
**Kuga-gun, Yamaguchi-ken (JP)**

(74) Representative: **Myerscough, Philip Boyd et al, J.A.Kemp & Co. 14, South Square Gray's Inn London, WC1R 5EU (GB)**

Courier Press, Leamington Spa, England.

Process for separation of alkylphenols by azeotropic distillation

This invention relates to a process for the separation of at least two alkyl phenols having close boiling points by the azeotropic distillation of a mixture composed of the alkyl phenols of a hydrocarbon azeotroping agent. Specifically, this invention relates to a process for separating 2,6-xylenol and o-cresol by the azeotropic distillation of a crude 2,6-xylenol which contains o-cresol with commercial advantage and an improved separating efficiency without the likelihood of coloration attributable to degeneration of the azeotroping agent and of reduction of the quality of the separated compounds.

Various methods such as distillation, crystallization, extraction, adsorption or combinations of these have been suggested for the separation of a mixture of cresol isomers, a mixture of cresols and xylenols, and mixtures of alkyl phenols including these. Of the exemplified methods, crystallization, extraction and adsorption are not good commercial methods because the separating procedure is complicated, and the efficiency of separation is low. In separating a higher-boiling alkyl phenol and a lower-boiling alkyl phenol from a mixture of at least two alkyl phenols having close boiling points such as a mixture of o-cresol, m-cresol and p-cresol, or a mixture of methylated products of cresol or phenol including phenol, o-cresol, p-cresol, 2,4-xylenol and 2,6-xylenol, a rectifying column having a number of theoretical trays is required, and the distillation should be carried out at high reflux ratio. In addition to these disadvantages, such a separating procedure also has disadvantages in regard to the amount of heat required and the rate of distillation.

In an attempt to overcome these disadvantages, US-A-3,397,124 discloses a process for the separation of at least two alkyl phenols having close boiling points by azeotropic distillation with an alkene as an azeotroping agent, especially a branched higher alkene having 10 to 14 carbon atoms and a boiling point within 30°C of more polar alkyl phenol in said alkyl phenols. This process is limited to the use of alkenes which are unsaturated hydrocarbons, and the use of the trimerization product of methylpropene (isobutylene) having a boiling point of about 175 to 177°C is recommended.

We have discovered that the azeotroping agent used in US-A-3,397,124, presumably because of its unsaturated bond, is degenerated and colored relatively easily during the azeotropic distillation procedure and builds up in the distillation bottom which contains alkyl phenols having higher-boiling points, thus making it difficult to obtain these higher-boiling alkyl phenols of high quality, and that this problem becomes more pronounced when an attempt is made to perform the distillation of the lower-boiling alkyl phenols fully from the top of the distillation tower. It was also found that there is still room for improvement in the content of lower boiling alkyl phenols in the azeotrope from the top, and the separating efficiency is unsatisfactory.

US-A-3,337,424 describes the purification of m- and p-cresol by the azeotropic distillation of crude mixtures containing them, and the recovery of the desired m,p-cresols from an azeotropic distillate. US-A-3,337,424 does not, however, describe an azeotropic separation of o-cresol from a phenolic mixture containing it, and the azeotroping agents whose use is illustrated therein have not been found to be effective in the azeotropic separation of 2,6-xylenol and o-cresol.

The present invention provides a process wherein 2,6-xylenol and o-cresol are separated by the azeotropic distillation of a crude 2,6-xylenol which contains o-cresol, which crude 2,6-xylenol has been obtained by the methylation of phenol or cresol, using, as azeotroping agent, n-decane, a mixture of dodecane isomers having a boiling point within the range of 150 to 190°C at 760 mm Hg (101.325 kPa), 2,6-dimethyloctane or 2,2,4,6,6-pentamethylheptane, o-cresol being removed in the distillate and 2,6-xylenol being left in the residue.

The process of the invention provides improved separating efficiencies without coloration problems.

o-Cresol has a boiling point of about 191°C, while a mixture of o-cresol and 2,6-xylenol used as starting material has a boiling point of about 201°C. The mixture of 2,6-xylenol and o-cresol used as starting material may contain other higher-boiling alkyl phenols or phenols.

The azeotroping agents used in accordance with the invention are all saturated aliphatic hydrocarbons which have a boiling point of 150 to 190°C at 760 mmHg. Thus 2,6-dimethyloctane has a boiling point of 159°C, n-decane a boiling point of 174°C, the mixture of dodecane isomers has a boiling range of 150 to 190°C, and 2,2,4,6,6-pentamethylheptane has a boiling point of 180°C. These azeotroping agents may be used singly or as a mixture of two or more.

The amount of the azeotroping agent can be selected as desired and can be, for example, 100 to 5,000 parts by weight, preferably 400 to 2,000 parts by weight, per 100 parts by weight of the crude 2,6-xylenol.

The distillation temperature in the process of the invention may be any suitable temperature at which the o-cresol azeotropes, for example a temperature of 150 to 190°C at atmospheric pressure.

The azeotropic distillation can be carried out under atmospheric, reduced or elevated pressures. Most commonly, the distillation is carried out at atmospheric pressure. Reduced pressures of up to 50 mmHg, and elevated pressures of up to 5000 mmHg can also be employed.

Distillation may be carried out continuously or batchwise. Separation of the o-cresol from the resulting azeotrope (top) can be performed by a customary means such as steam distillation or crystallization.

The following Examples and Comparative Examples illustrate the present invention.

### Example 1

n-Decane ($C_{10}$) (113.6 g) was added to 86.4 g of crude 2,6-xylenol containing 13% by weight of o-cresol. The mixture was put into a 300 ml round-bottomed flask, and distilled at atmospheric pressure in an Oldershaw-type distillation column having 10 trays. Distillation began at 160°C. Ten milliliters of the initial fraction was isolated and analyzed by gas chromatography. The results are shown in Table 1.

No degeneration occurred in n-decane after heat-treatment at 180°C for 6 hours under reflux.

### Example 2

Distillation was carried out under the same conditions as in Example 1 except that n-decane was replaced by dodecane isomers ($C_{12}$). The results are shown in Table 1.

The dodecane isomers were prepared as follows: To 200 g of reagent triisobutylene (a product of Tokyo Chemical Co., Ltd.) was added 20 g of 2%Pd/$Al_2O_3$, and the triisobutylene was hydrogenated at 150°C under atmospheric pressure. The product had a boiling point of 177°C at 760 mmHg.

No degeneration occurred in the dodecane isomers after heat-treatment at 180°C for 6 hours under reflux.

### Comparative Examples 1, 2 and 3 Distillation was carried out under the same conditions except that n-octane ($C_8$), n-undecane ($C_{11}$; b.p. about 196°C) or triisobutylene ($C_{12}$ unsaturated) was used instead of the n-decane.

The triisobutylene was degenerated and colored (Hazen color: 140 APHA) after heat-treatment at 180°C for 6 hours under reflux.

### Examples 3 and 4

Distillation was carried out under the same conditions except that 2,6-dimethyloctane ($C_{10}$) or 2,2,4,6,6-pentamethylheptane ($C_{12}$) was used instead of n-decane. The results are shown in Table 1.

The 2,6-dimethyloctane or 2,2,4,6,6-pentamethylheptane was not degenerated after heat-treatment at 180°C for 6 hours under reflux.

TABLE 1

| | Azeotroping agent | | Composition of the starting mixture (wt. %) | | | Distillation temperature at the top (°C) | Composition of the distillate (wt. %) | | | |
|---|---|---|---|---|---|---|---|---|---|---|
| | Type | Boiling point (°C at 760 mmHg) | Additive | o-Cresol | 2,6-Xylenol | | Additive | o-Cresol | 2,6-Xylenol | APHA (*1) |
| Example 1 | n-Decane | 174 | 56.8 | 5.6 | 37.6 | 165 | 87.2 | 10.6 | 2.1 | 10 |
| Example 2 | Dodecane isomers | 177 | 56.5 | 5.6 | 37.9 | 171 | 81.3 | 15.2 | 3.5 | 10 |
| Comparative Example 1 | n-Octane | 126 | 56.5 | 5.6 | 37.9 | 126 | 99.6 | 0.3 | less than 0.1 | — |
| Comparative Example 2 | n-Undecane | 195.9 | 56.5 | 5.6 | 37.9 | 181 | 59.9 | 24.9 | 15.2 | — |
| Comparative Example 3 | Triiso-butylene | 177 | 60.5 | 5.2 | 34.3 | 170 | 89.9 | 7.8 | 2.3 | 140 |
| Example 3 | 2,6-Dimethyl-octane | 159 | 56.5 | 5.6 | 37.9 | 155 | 90.6 | 8.4 | 1.0 | 10 |
| Example 4 | 2,2,4,6,6-Pentamethyl-heptane | 180 | 56.5 | 5.6 | 37.9 | 174 | 79.5 | 16.4 | 4.1 | 10 |

(*1): ASTM D—1209 (Hazen color).

## Claims

1. A process for separating at least two alkyl phenols having close boiling points by the azeotropic distillation of a mixture composed of the alkyl phenols with a hydrocarbon azeotroping agent, characterised in that 2,6-xylenol and *o*-cresol are separated by the azeotropic distillation of a crude 2,6-xylenol which contains *o*-cresol, which crude 2,6-xylenol has been obtained by the methylation of phenol or cresol, using, as azeotroping agent, n-decane, a mixture of dodecane isomers having a boiling point within the range of 150 to 190°C at 760 mm Hg (101.325 kPa), 2,6-dimethyloctane or 2,2,4,6,6-pentamethylheptane, *o*-cresol being removed in the distillate and 2,6-xylenol being left in the residue.

2. A process according to claim 1 wherein the azeotropic distillation is carried out at atmospheric pressure.

## Revendications

1. Procédé de séparation d'au moins deux alkyl phénols ayant des points d'ébullition voisins par distillation azéotropique d'un mélange composé desdits alkyl phénols et d'un hydrocarbure utilisé comme agent azéotropique, caractérisé par le fait que l'on sépare le 2,6-xylénol et l'*o*-crésol par distillation azéotropique d'un 2,6-xylénol brut qui contient de l'*o*-crésol, ledit 2,6-xylénol brut ayant été obtenu par la méthylation de phénol ou de crésol, en utilisant comme agent azéotropique, le n-décane, un mélange d'isomères de dodécane ayant un point d'ébullition dans la gamme de 150 à 190°C sous 760 mmHg (101,325 kPa), le 2,6-diméthyloctane ou 2,2,4,6,6-pentaméthyl-heptane, l'*o*-crésol étant éliminé avec le distillat et le 2,6-xylénol restant dans le résidu.

2. Procédé selon la revendication 1, caractérisé par le fait que la distillation azéotropique est effectuée sous la pression atmosphérique.

## Patentansprüche

1. Verfahren zur Trennung von mindestens zwei Alkylphenolen mit nahe beieinander liegenden Siedepunkten durch azeotrope Destillation eines Gemisches, bestehend aus den Alkylphenolen und einem azeotrop siedenden Kohlenwasserstoff, dadurch gekennzeichnet, daß 2,6-Xylenol und o-Kresol getrennt werden durch azeotrope Destillation eines rohen 2,6-Xylenols, das o-Kresol enthält, wobei das rohe 2,6-Xylenol erhalten worden ist durch Methylierung von Phenol oder Kresol, wobei als azeotropes Mittel n-Decan, ein Gemisch von Dodecanisomeren mit einem Siedepunkt im Bereich von 150 bis 190°C bei 760 mm Hg (101,325 kPa), 2,6-Dimethyloctan oder 2,2,4,6,6-Pentamethylheptan verwendet wird, und wobei o-Kresol im Destillat entfernt wird und 2,6-Xylenol im Rückstand verbleibt.

2. Verfahren nach Anspruch 1, wobei die azeotrope Destillation bei Atmosphärendruck durchgeführt wird.